Europäisches Patentamt

European Patent Office

Office européen des brevets

(11) Publication number: **0 188 312**
A2

# EUROPEAN PATENT APPLICATION

(21) Application number: 86300042.8

(22) Date of filing: 06.01.86

(51) Int. Cl.⁴: **C 12 N 15/00**
C 12 P 21/02, C 07 K 13/00
A 61 K 37/64, C 12 N 9/99

(30) Priority: 07.01.85 GB 8500341
01.11.85 GB 8526951

(43) Date of publication of application:
23.07.86 Bulletin 86/30

(84) Designated Contracting States:
AT BE CH DE FR GB IT LI LU NL SE

(71) Applicant: CELLTECH LIMITED
244-250 Bath-Road
Slough Berkshire SL1 4DY(GB)

(72) Inventor: Harris, Timothy John Roy
54 Red Rose
Binfield Bracknell Berkshire(GB)

(72) Inventor: Docherty, Andrew James Penrose
34 Seymour Road
Hampton Hill Middlesex TW12 1DD(GB)

(72) Inventor: Reynolds, John James
6 The Biggen
Duxford Cambridgeshire CB2 4SQ(GB)

(72) Inventor: Murphy, Gillian
204 Cambridge Road
Great Shelford Cambridgeshire, CB2 5UV(GB)

(74) Representative: Votier, Sidney David et al,
CARPMAELS & RANSFORD 43, Bloomsbury Square
London WC1A 2RA(GB)

(54) Process for the production of a protein.

(57) A process for producing a metalloproteinase inhibitor or a precursor thereof comprising culturing host cells transformed with a vector including a gene coding for the metalloproteinase inhibitor or the precursor and, if appropriate, cleaving the precursor to yield metalloproteinase inhibitor. The precursor may be a premetalloproteinase inhibitor or a fusion protein comprising a heterologous protein and a metalloproteinase inhibitor. The metalloproteinase inhibitor has application as the effective component in a pharmaceutical composition for the treatment of disease processes where accelerated breakdown of the extracellular organic matrix is observed.

This invention relates to a process for the production of a protein, particularly a metalloproteinase inhibitor, by a recombinant DNA process. The invention also relates to specific proteins, genes, vectors, host organisms and to pharmaceutical compositions.

The accelerated, uncontrolled breakdown of connective tissues by enzymic resorption of the extracellular organic matrix is a feature of many pathological conditions (D.E. Woolley (1984) Mammalian Collagenases pp. 119-157 In: Extracellular Matrix Biochemistry, Eds. K.A. Piez and A.H. Reddi, Elsevier, New York).

Enzymic resorption occurs in both normal and diseased states and is due, in part, to the activity of several metalloproteinases - the most important one of which is thought to be collagenase (Sellers and Murphy (1981) International Review of Connective Tissue Research, 9, 151-190). Normally the activity of these catabolic enzymes is tightly regulated as illustrated by the control of collagen breakdown in the uterus postpartum (Woessner (1979) Biochem. J., 180, 95-102 and Woessner (1980) In: Collagenase in Normal and Pathological Connective Tissues, Eds. D.E. Wooley and J.M. Evanson 223-239 Wiley New York) and by the close coupling of collagen degradation and synthesis in bone remodelling (Raisz et al (1978) In: Mechanisms of Localised Bone Loss Eds. J.E. Horton, T.M. Tapley and W.F. Davis 39-45. Information Retrieval Inc., Washington, D.C. and London). However in, for example, rheumatoid arthritis and oesteoarthritis, uncontrolled collagen degradation results in irreversible mechanical failure of the connective tissue (Kempson _et al_ (1976) Biochim. Biophys. Acta., _428_, 741-760).

It has been suggested that the strict control of collagenase activity is brought about by a potent inhibitor (Reynolds _et al_ (1977) The Lancet _2_, 333-335). Inhibitors of collagenase have been detected in the culture media of connective tissues (Murphy _et al_ (1977) Biochim. Biophys. Acta., 483, 493-498, Shinkai _et al_ (1977) J. Biochem. (Tokyo) _81_, 261-263, Vater _et al_ (1979) J. Biol. Chem. _254_, 3046-3053, Nolan _et al_ (1980) Atherosclerosis _35_, 93-102, Yasui _et al_ (1981) Collagen Res. _1_, 59-72, Dean and Woessner (1984) Biochem. J. _218_, 277-280) in cells (Nolan _et al_ (1978) Biochem. Biophys. Res. Comun. _83_, 1183-1190, Welgus _et al_ (1979) J. Biol. Chem. _254_, 1938-1943, Kerwar _et al_ (1980) Biochem. Biophys. Acta. _632_, 183-191, Pettigrew _et al_ (1980) Arch. Oral Biol. _25_, 269-274, Sapolsky _et al_ (1981) Biochim. Biophys. Acta. 658, 138-147, Macartney and Tscheshe (1983) Eur. J. Biochem. _130_, 79-83, Stricklin and Welgus (1983) J. Biol. Chem. _258_: 12252-12258, McGuire-Golding _et al_ (1983) Biochim. Biophys. Acta. _763_, 129-239) in serum (Woolley _et al_ (1976) Nature (London) _262_, 353-327, Woolley _et al_ (1978) Biochim. Biophys. Acta. _522_, 205-217) and in other body fluids (Bunning _et al_ (1984) Eur. J. Biochem. _139_, 75-80). In addition, the collagenase inhibitor from cultured rabbit bone was shown to inhibit two other neutral metalloproteinases which degrade gelatin and proteoglycan (Sellers _et al_ (1979) Biochem. Biophys. Res. Commun. _87_, 581-587). This inhibitor, tissue inhibitor of metalloproteinase, (hereinafter referred to as 'TIMP') and another inhibitor isolated from human amniotic fluid, were purified and characterised as very similar proteins of approximate molecule weight 28,000 (Cawston et al (1981) Biochem. J. 195, 167-170)   It now seems likely that all the above mentioned inhibitors are related to TIMP (Hembry et al, (1985), J. Cell Sci., 73, 105-119).

The ubiquitous presence of TIMP in most tissues and cells in culture (Reynolds et al, (1981), In: Research Monographs in Cell and Tissue Physiology: Cellular Interactions, 6, Eds. J.T. Dingle and J.L. Gordon 205-215.   Elsevier/North Holland Biochemical Press, Amsterdam) and its ability to form a tight complex with either active collagenase or active proteoglycanase (Cawston et al, (1983), Biochem. J., 211, 313-318) indicates that all connective tissues synthesise TIMP as a 'fail safe' mechanism to control the local activity of various catabolic enzymes (Reynolds et al, (1977), The Lancet, 2, 333-335; Murphy and Sellers (1980) In: Collagenase in Normal and Pathological Connective Tissues, Eds. D.E. Wooley and J.M. Evanson 65-81, John Wiley, London; Sellers and Murphy (1981), In: International Rev. of Connective Tissue Res., 9, 151-190 Eds. D.A. Hall and D.E. Jackson, Academic Press Inc., New York).   For example, the results of studies with human joint tissue from normal and arthritic subjects show similarities with results from a rabbit model which support the proposal that resorption is closely linked with insufficient TIMP to counteract increases in local metalloproteinase production (McGuire et al, (1981), Clinical Sci., 703-710; Murphy et al, (1981), Clinical Sci., 61, 711-716; Cambray et al, (1981), Rheumatol. Int., 1, 11-16; Murphy et al, (1981), Rheumatol. Int., 1, 17-20).

Furthermore, there is evidence that the invasiveness of tumours is inversely related to the level of TIMP in the surrounding matrix (Malaka et al, (1983), J. Neurosurg., 59, 461-466; Hicks et al, (1984), Int. J. Cancer, 33, 835-844).  Finally, in an experimental tumour invasion model (Thorgeirsson et al, (1982), J.N.C.I., 69, 1049-1054) it was found that a purified metalloproteinase inhibitor was essentially the only inhibitor effective in preventing tumour invasion.

TIMP could be of value in blocking those disease processes where accelerated breakdown of the extracellular organic matrix is observed.  However, further evaluation of the therapeutic usefulness of TIMP and, indeed, its commercial value as a therapeutic agent has been prevented by the lack of material from natural sources.  For example, only 16.4 mgs of protein were purified from 70 litres of human skin fibroblast cell culture supernatant (Stricklin and Welgus, (1983), J. Biol. Chem., 258, 12252-12258).

According to the present invention there is provided a process for the production of a metalloproteinase inhibitor comprising culturing host cells transformed with a vector including a gene coding for the metalloproteinase inhibitor.

The process allows the production of relatively large quantities of metalloproteinase inhibitor and, for the first time, facilitates complete characterisation of the structure and pharmacological properties of the protein.

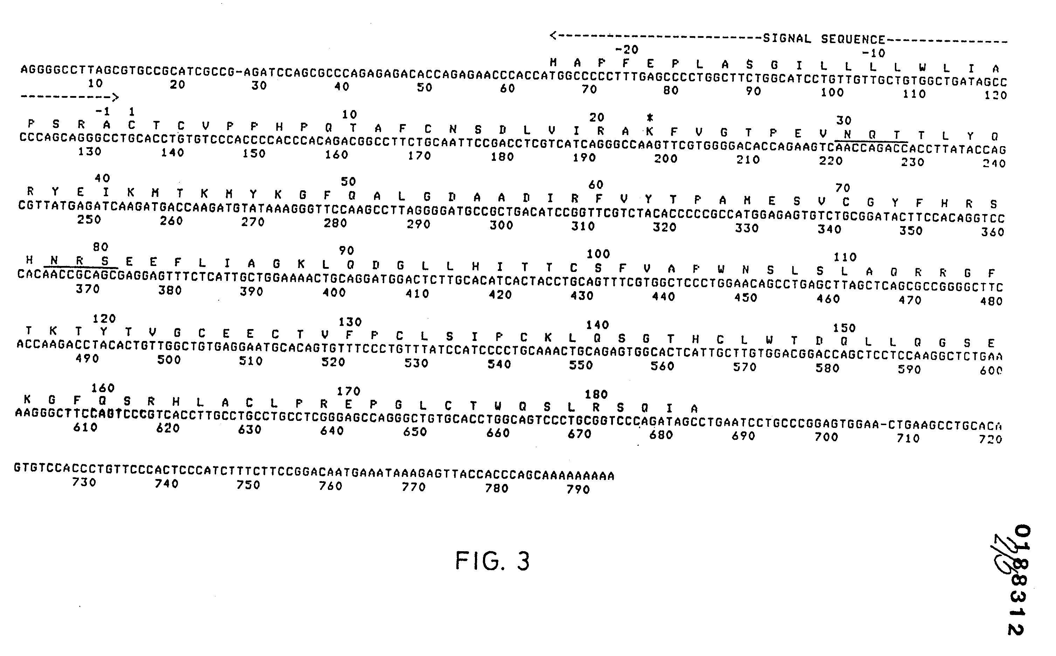

The metalloproteinase inhibitor is a protein capable of substantially reducing the activity of one or more metalloproteinases.  Preferably however the inhibitor is capable of reducing the activity of those metalloproteinases which are active during extracellular matrix resorption, e.g. collagenase. Most preferably the inhibitor is tissue inhibitor of metalloproteinase (TIMP). Several TIMP-like proteins have been described in the literature but their similarities suggest that they are closely related, if not identical to TIMP. Preferably therefore, the tissue inhibitor of metalloproteinase has an amino acid sequence of greater than 90 percent homology (common amino acids/total amino acids) with the sequence of amino acids 1 to 184 as shown in Figure 3 of the accompanying drawings. More preferably, the homology is greater than 98 percent and most preferably the tissue inhibitor of metalloproteinase has the amino acid sequence substantially as shown in Figure 3.

In addition to complete metalloproteinase inhibitor proteins, it is envisaged that relatively small peptide fragments of such proteins e.g. a peptide fragment of TIMP, may be useful as metalloproteinase inhibitors. Thus, in a particular embodiment of the first aspect of the invention, and where appropriate in subsequent aspects of the invention, the term 'metalloproteinase inhibitor' includes a metalloproteinase inhibiting peptide fragment of a metalloproteinase inhibitor protein.

The metalloproteinase inhibitor is suitably a methionine-metalloproteinase inhibitor. It is currently understood that to obtain expression of a gene, the gene must possess a 5' ATG codon and the corresponding polypeptide therefore possesses an N-terminal methionine amino acid. As used herein the term "methionine-metalloproteinase inhibitor" denotes an authentic mammalian metalloproteinase inhibitor (or an authentic mammalian metalloproteinase inhibitor, modified or substituted to provide a functionally equivalent protein) having an N-terminal methionine residue. Preferably the methionine residue is adjacent the N-terminal amino acid of the metalloproteinase inhibitor but may be separated therefrom by one or more amino acids provided that the protein possesses metalloproteinase inhibitor functional activity.

In a further aspect of the invention there is provided a process for the production of a metalloproteinase inhibitor comprising producing a precursor of the metalloproteinase inhibitor by culturing host cells transformed with a vector including a gene coding for the metalloproteinase inhibitor precursor and cleaving the precursor to produce the metalloproteinase inhibitor.

The precursor protein may be a methionine-metalloproteinase inhibitor.

The precursor protein may be a metalloproteinase inhibitor with an amino terminal signal sequence. The signal sequence may be a sequence having the effect of encouraging transport of the expression products from the host cell in which the genes have been expressed. For example, in the present case, a signal sequence of 23 amino acids as depicted by amino acids -1 to -23 in Figure 3 of the accompanying drawings may be attached to the amino terminal of the metalloproteinase inhibitor to form a premetalloproteinase inhibitor. This signal sequence assists in the export of the product from eukaryotic host cells and is itself cleaved from the product during transport through the cell wall.

The precursor protein may be a fusion protein comprising a heterologous protein and a metalloproteinase inhibitor protein.    The heterologous protein may be all or a part of a protein capable of being produced in a host organism, preferably at a high level.    Two such heterologous proteins are β-galactosidase and the product of the trpE gene.    The fusion protein preferably includes a site susceptible to selective chemical or enzymic cleavage between the metalloproteinase inhibitor protein and the heterologous protein.    The heterologous protein may be a yeast signal sequence and the host organism may be a yeast.    In this preferred embodiment, the yeast host organism advantageously cleaves the fusion protein to produce a mature metalloproteinase inhibitor.

In a further aspect of the invention, we provide a metalloproteinase inhibitor produced by the process of the first aspect of the invention or a metalloproteinase inhibitor precursor protein produced as an intermediate compound.

In a further aspect of the invention, we provide a fusion protein comprising a heterologous protein and a metalloproteinase inhibitor.

In a further aspect of the invention, we provide a gene coding for the amino acid sequence of a metalloproteinase inhibitor, or a precursor thereof.    Preferably, we provide a gene having substantially the nucleotide sequence from 64 to 684 inclusive as shown in Figure 3 of the accompanying drawings.    Preferably we provide a gene including the nucleotide sequence from 133 to 684 inclusive as shown in Figure 3 of the accompanying drawings.

In a further aspect of the invention, we provide a vector including a gene as defined above. The vector is adapted for use in a given host cell by the provision of suitable selectable markers, promoters and other control regions as appropriate.

In a further aspect of the invention, we provide host cells transformed with a vector according to the invention. The host cells may be any host organism which may be transformed with a vector including a gene coding for a metalloproteinase inhibitor or a precursor thereof, such that expression of the gene occurs. Suitable host cells include yeasts (for example, Saccharomyces cerevisiae) and mammalian cells in tissue culture (for example, hamster ovary or mouse mammary tumour cells). Preferably, where the host cell is a bacterium or a yeast, the vector includes a gene coding for a methionine-metalloproteinase inhibitor or a fusion protein including a metalloproteinase inhibitor and when the host cell is a mammalian cell in tissue culture, the vector preferably includes a gene coding for a premetalloproteinase inhibitor.

In a further aspect of the invention, we provide a pharmaceutical composition comprising a metalloproteinase inhibitor and a pharmaceutically acceptable excipient. The pharmaceutical composition may be in the form of an injectable solution.

We also provide a process for the production of a pharmaceutical composition comprising bringing a metalloproteinase inhibitor into association with a pharmaceutically acceptable carrier.

Preferably, the metalloproteinase inhibitor produced in accordance with the invention will have a useful pharmacological effect without significant antigenic reaction with the immune system.    In particular the compounds may be used in the treatment of cancer by prevention of tumour cell invasion or tumour vascularisation.    The compounds may also be used for treating skin disease such as the various forms of epidermolysis bullosa, periodontal disease, scleroderma and also the soft tissue destruction in cutaneous neoplasia.

Furthermore, the connective tissue destruction associated with the various forms of arthiritis such as ulceration of the cornea and destruction of joint tissues in rheumatoid arthritis may also be treated with the compounds.    In addition, it has been reported (Docherty et al, (1985) Nature, 318, 66-69) that a protein identical in structure to TIMP may have erythroid potentiating  activity and thus the compounds may have therapeutic use in the treatment of disorders of the haematopoetic system.

Various aspects of the invention are now exmplified by the following drawings in which:

Figure 1 - shows the N-terminal amino acid sequences of human foetal lung fibroblast and human amniotic fluid TIMP, (a), and human skin fibroblast collagenase inhibitor, (b), together with a 69 base oligonucleotide probe, (c), and a complementary 18-mer, (d),

Figure 2 - shows a restriction map of human TIMP cDNA compiled from one almost full length cDNA and the 5' end (broken line) of one additional overlapping cDNA,

Figure 3 - shows the nucleotide sequence
of human TIMP cDNA and the predicted
amino acid sequence,

Figure 4 - shows a restriction map of the
E.coli expresson vector pMG454
(The closed boxed represents a
61 bp linker),

Figure 5 - shows a restriction map of the
mammalian cell expression vector
pTIMP-1,

Figure 6 - shows a 12% SDS polyacrylamide
gel indicating Coomassie blue
stained proteins made in E.coli
E103S transformed with pMG454
(Lane 1: marker proteins, lane 2:
non-induced control cells harbouring the expression vector with no
TIMP cDNA insert, lane 3: induced
control cells, lane 4: non-induced
E103S cells harbouring pMG454,
lane 5: as lane 4 but induced,
lane 6: purified E.coli β-
lactamase),

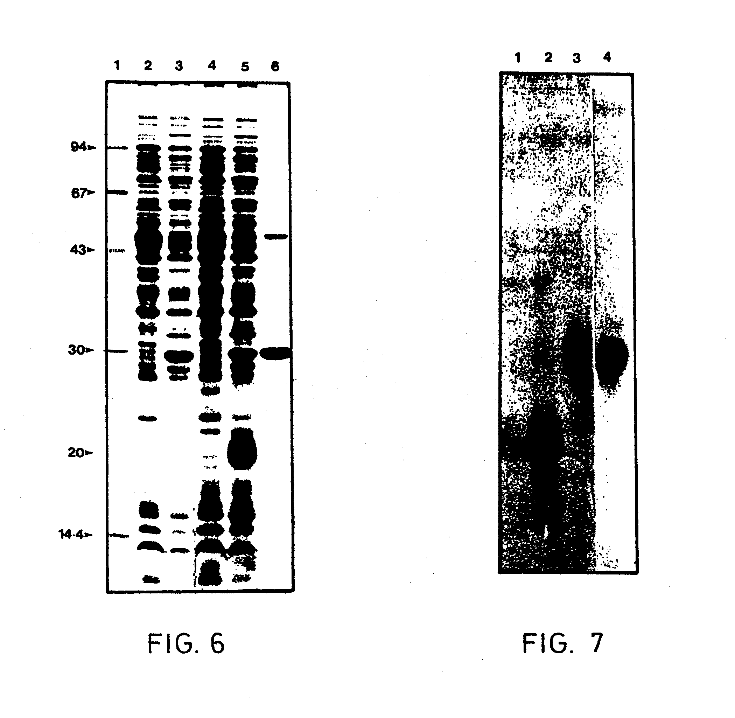

Figure 7 - shows an autoradiograph obtained
after Western blotting with a
TIMP antibody (Lanes 1 and 2: as
lanes 4 and 5 in Figure 6, lane
3: purified human amniotic fluid
TIMP, lane 4: culture supernatant
proteins from a C127 cell line
transfected wtih pTIMP-1) and

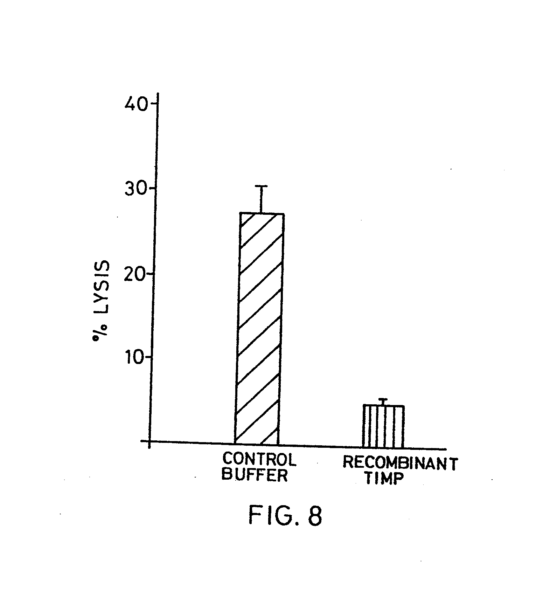

Figure 8 - is a bar chart showing the
inhibition by recombinant TIMP
of lysis of collagen films caused
by rabbit tumour cells.

METHOD

1. THE ISOLATION OF A HUMAN cDNA ENCODING TIMP

1.1 Purification and N-terminal sequencing of human TIMP

Human foetal diploid lung cells (ATCC CLL153) purchased at passage 13 (Flow laboratories) were maintained in Dulbecco's modified Eagles medium (DMEM; Gibco, Europe Ltd.) supplemented with 4mM glutamine, 15% heat inactivated fetal calf serum (FCS) and 100 IU ml penicillin and 100 $\mu$g ml$^{-1}$ streptomycin. Cells were expanded into two 490 cm$^2$ plastic roller bottles, allowed to reach confluence in growth medium, washed with serum free DMEM and then maintained in serum free media for 6 days. Culture supernatant TIMP was measured by inhibition of rabbit skin collagenase using the collagen fibril assay (Murphy, Cawston and Reynolds, J. Biochem., 195, 167-170, (1981). One unit of TIMP inhibits 2 units of collagenase (2 $\mu$g of collagen degraded min$^{-1}$) by 50%. TIMP levels varied from less than 0.2 U to 5 U/10$^5$ cells/48 h and low producing cells were stimulated by the addition of 5 ng ml$^{-1}$ 4$\beta$-phorbol-12$\beta$-myristate 13$\alpha$-acetate (PMA) to the serum free culture medium (Murphy, Reynolds and Werb, (1985), J. Biol. Chem., 260, 3079-3083). Culture supernatants and cells were harvested after 6 days, the former being concentrated 40-fold with an Amicon concentrater using a Diaflo hollow fibre cartridge with a $M_r$ 10,000 cut-off.

TIMP was purified from the concentrated supernatants as described by Murphy, Cawston and Reynolds (J. Biochem., 195, 167-170, (1981)) and reduced and carboxymethylated by standard procedures. TIMP from human amniotic fluid was similarly purified, reduced and carboxymethylated.

NH$_2$-terminal sequence was obtained by automated Edman degradation on an Applied Biosystems gas phase sequenator. A comparison of these N-terminal sequences with that published for a collagenase inhibitor (Stricklin and Welgus, J. Biol. Chem., 258, 12252-12258, (1983)), with properties similar to TIMP by human skin fibroblasts (Welgus and Stricklin, J. Biol. Chem., 258, 12259-12264) is shown in Figure l(a). Human amniotic fluid and human foetal lung TIMP are identical for all 28 N-terminal amino acids identified. With the exception of a leucine, (instead of lysine at position 22) the human skin fibroblast inhibitor is also identical for 23 N-terminal residues. (Figure (1(b)).

Based on this information, a single 69-base oligonucleotide probe capable of encoding the 23 N-terminal amino acids of TIMP was designed (Lathe, J. Mol. Biol., 183, 1-12 (1985)) (Figure l(c)). The codons were those reported to appear most frequently in human genes except in three positions where to have done so would have incorporated the rarely observed dinucleotide, CpG (Grantham et al, Nuc. Acid. Res., 9, r43-47, (1981); Nussenov, J. Mol. Biol., 149, 125-131, (1981). In addition, an 18mer complementary to nucleotides 25-42 of the long probe was also constructed (Fig. l(d)). The 69-mer oligonucleotide probe was synthesised on an Applied Biosystems model 380A synthesiser by monomer addition of activated phosphoamidites to a solid support. The deprotected probe was purified by HPLC. The 18mer was synthesised by automated solid phase phosphotriester chemistry (Patel et al, Nuc. Acid Res., 10, 5605-5619, (1982)) and also purified by HPLC. The numbers above the probe sequence

- 13 -

0188312

refer to nucleotide sequence of the cDNA Figure 3. The letters above the probe sequence represent those nucleotides found to be different in the cDNA.

## 1.2 Preparation of antibody to TIMP

Sheep anti human amniotic fluid TIMP serum was provided by R. Hembry (Strangeways Research Laboratories, Cambridge CB1 4RN, United Kingdom). Its preparation has been described (Hembry et al, (1985), J. Cell Sci., 73, 105-119).

Some experiments employed immunoglobulin (IgG) which was precipitated from the sheep antiserum with solid sodium sulphate (18% w/v), redissolved in phosphate buffered saline (PBS) and dialysed extensively against the same buffer. The resulting IgG preparation was diluted 100-fold in 10mM Tris HCl pH 7.6, 160 mM NaCl, 0.5% w/v casein and 0.05% v/v Tween 20. It was then absorbed against E.coli DH1 antigens by incubating with nitrocellulose filters (Schleicher and Schull) on which confluent lawns of E.coli colonies had been lysed with urea as described by Docherty et al ((1985), Nuc. Acid Res., 13, 1891-1903). After overnight incubation at 4°C, the IgG preparation was clarified by centrifugation and then stored at -20°C before use.

## 1.3 mRNA Preparation and In Vitro translation

Human foetal diploid lung cells (ATCC CCL153) were grown as described for protein purification. After six days in serum free media, cell monolayers were washed and then harvested from the roller bottles as described by Maniatis et al ((1982) In: Molecular Cloning: A Laboratory Manual, 191, Cold Spring Harbor Laboratory, New York).

DNA was extracted from the cells using the guanidinium isothiocyanate phenol procedure (Maniatis et al, (1982), In: Molecular Cloning: A Laboratory Manual, 194-195, Cold Spring Harbor Laboratory, New York). Polyadenylated mRNA was isolated from the total RNA by oligo dT-cellulose (Collaborative Research) column chromatography (Aviv and Leder (1972), Proc. Nat. Acad. Sci. USA, 69, 1408-1412) and its integrity then checked by agarose gel electrophoresis and in vitro translation in a rabbit reticulocyte lysate in the presence of $^{35}$S methionine (Pelham and Jackson, (1976), Eur. J. Biochem., 67, 247-256). Immunoprecipitation of the in vitro translation products was next undertaken (Harris et al, (1981), Virology, 112, 91-98) using the sheep antiserum to human TIMP and protein A sepharose to precipitate the complexes. Analysis of the peptides on a 10% polyacrylamide gel (Laemmli, (1970), Nature London, 227, 680-685) revealed that a single peptide of approximate molecular weight 23,000 had been immunoprecipitated with the TIMP antibody. This indicated the presence of TIMP mRNA in the mRNA isolated from the CCL 153 cell line. Although the yield of the $M_r$ 23,000 polypeptide varied after translation of mRNA from various tissue culture experiments all the mRNA preparations were pooled and used in the subsequent cDNA synthesis.

## 1.4 cDNA Synthesis

The mRNA was used to synthesise single stranded cDNA by reverse transcriptase (Anglian Biotechnology Limited) essentially as described by Retzel et al (Biochemistry, (1980), 19, 513-518). It was then converted to double stranded cDNA by using E.coli DNase-H (Enzo Biochem), DNA polymerase (Boehringer) and E.coli DNA ligase (Boehringer) (see Gubler and Hoffman, (1983), 25, 263-269). Terminal deoxynucleotidyl transferase (Miles) was then used to catalyse the stepwise addition of dCTT to the 3' OH termini of the cDNA essentially as recommended by Michelson and Orkin (J. Biol. Chem., (1982), 257, 14773-14782). Varying amounts of the dCTP tailed DNA were then annealed with dGTP tailed pBR322 plasmid DNA (BRL, Lot No.21112) (Gubler and Hoffman (1983), 25, 263-269) before being used to transform E.coli DH1 (Manahan, (1983), J. Mol. Biol., 166, 557-580) to tetracycline resistance. In this way, conditions were established that gave rise to 50-100 tetracycline resistant transformants per ng of cDNA.

1.5 Screening the cDNA Library

A cDNA library of about 15,000-20,000 colonies arising from transformation of DH1 with the annealed DNAs was obtained. After growth at 37°C for 20 hours, replicas of the colonies were transferred to nitrocellulose filters (Schleicher and Schull) where their plasmid content was amplified by a further 16 hour incubation of the filters in the presence of 100 $\mu$g ml$^{-1}$ chloramphenical on LB-agar plates. In situ fixture of the DNA from the colonies to the nitrocellulose was then achieved as described by Maniatis et al (In: Molecular Cloning: A Laboratory Manual, 315, Cold Spring Harbor Laboratory, New York).

The nitrocellulose filters were then prehybridised at 65°C for 4 hours in a buffer consisting of 0.9M Tris HCl pH 7.4, 0.0005M EDTA, 0.5% v/v NP40, 0.2% w/v SDS, 100 $\mu$g ml$^{-1}$ sheared denatured salmon sperm DNA, 2 x Denhardts solution (Denhardt, (1966), Biochem. Biophys. Res. Commun., 23, 641-646). 2 mls of buffer were used per 13.5 cm diameter filter. After prehybridisation labelled 69mer probe DNA was added to a concentration of approximately 25 ng ml$^{-1}$. The probe had been labelled essentially as described by Maniatis et al (In: Molecular Cloning: A Laboratory Manual, 122, Cold Spring Harbor Laboratory, New York) except that unincorporated $^{32}$P ATP was removed by repeated precipitation with 2M ammonium acetate (Gubler and Hoffman, (1983), Gene, 25, 263-269). The specific activity of the labelled probe was 2.5 x 10$^5$ Cerenkov cpm pmol$^{-1}$ and the concentration of label during the hybridisation was 1.1 x 10$^6$ Cerenkov cpm ml$^{-1}$. After addition of the probe to the filters, the hybridisation temperature was reduced over a period of 3 hours to 42°C. Hybridisation at this temperature was continued for 12 hours.

The nitrocellulose filters were then washed in 0.5 x SSC (saline sodium citrate; 1 x SSC is 0.15M NaCl, 15 mM trisodium citrate pH 7.0) sequentially at 43°C for 45 minutes, 45°C for 30 minutes and 50°C for 15 minutes. The filters were finally rinsed in 0.5 x SSC at room temperature before being autoradiographed at -80°C for 20 hours with an intensifying screen. Ten putatively positive colonies were identified on the original plates which coincided with signals recorded by autoradiography. They were streaked for single colonies and subjected to a second round of oligonucleotide screening exactly as outlined above. Six of the streaked-out colonies again gave rise to positive signals. Clonally pure colonies from these streaks were therefore characterised further as follows.

## 1.6 Analysis of the Positive Clones

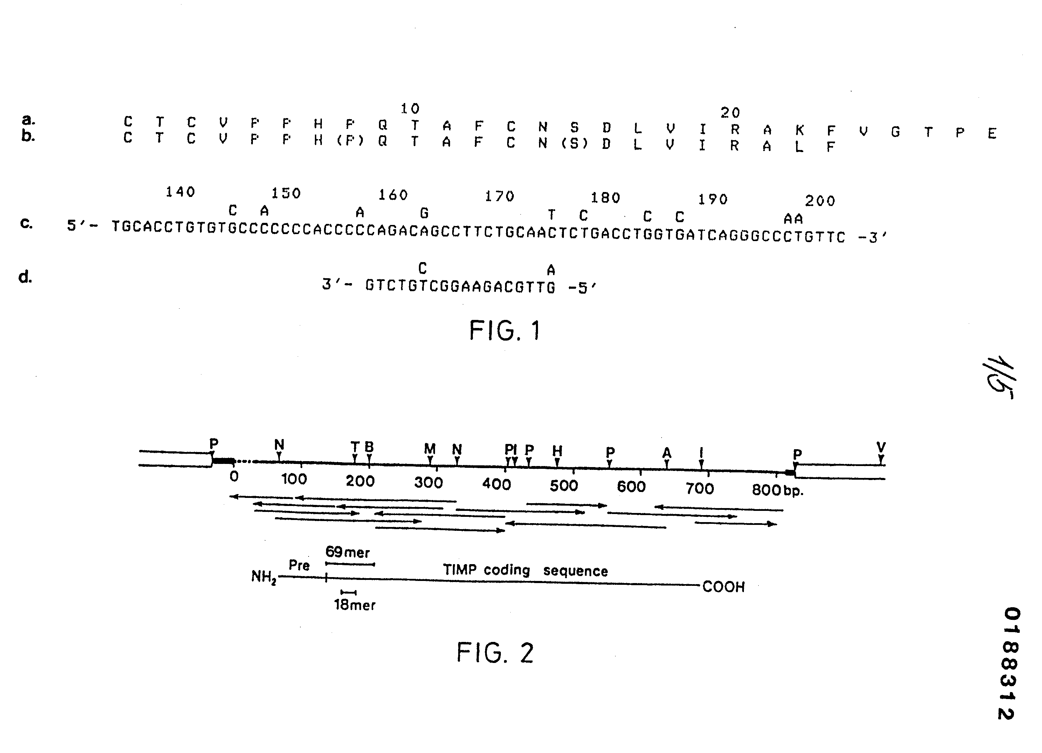

Plasmid DNA from the six positive clones was purified by the alkali lysis procedure followed by centrifugation in caesium chloride - ethidium bromide (Maniatis et al, (1982), In: Molecular Cloning: A Laboratory Manual, 90-94, Cold Spring Harbor Laboratory, New York). The relatedness of their cDNA inserts was then examined by direct sequencing of the uncut plasmid DNA essentially as described by Vieira and Messing (Gene, (1982), 19, 259-268) using the dideoxy procedure (Sanger et al, (1977), Proc. Nat. Acad. Sci. USA, 74, 5463-5467) with a primer complementary to nucleotides 25-42 of the 69mer used in the original screening (5-CTTGCAGAAGGCTGTCTG-3') synthesised by automated solid phase phosphotriester chemistry. (Patel et al, (1982), Nuc. Acid Res., 10, 5605-5619). This primer is therefore complementary to the sense strand which encodes residues 9-13 of mature TIMP (see Fig. 1). It was found that five of the six clones had identical DNA sequences over a region of at least seventy nucleotides and that clone 3 extended the furthest. Furthermore, by end labelling the 18mer (Maniatis et al, (1982), In: Molecular Cloning: A Laboratory Manual, 120, Cold Spring Harbor Laboratory, New York) before using it to prime sequencing of the clone 3 DNA, it was found that this DNA had the capacity to encode the first eight amino acids of the fibroblast inhibitor. This was taken as strong evidence that the clones isolated were cDNA clones for human TIMP.

The plasmid DNAs were then subjected to restriction analysis using restriction endonucleases under their recommended conditions (Amersham International). The resulting data permitted the represent pBR322 sequences and the thick line assembly of a restriction map which is shown in Figure 2. This map is compiled from clone 2 and the 5' end (broken line) of clone 3, the open boxes denotes the position of the GC tails. The arrows denote the directions of dideoxy sequencing. The following abbreviations are used; P = PstI, N = NcoI, T = TthIII-1, B = BstXI, M = MstII, A = AvaI, V = PvuI, H = HaeII, I = HinfI.

The complete nucleotide sequence compiled from clones 2 and 3 was obtained by the dideoxy method (Sanger et al, (1977), Proc. Natl. Acad. Sci. USA, 74, 5463-5467) after subcloning restriction fragments into M13 tg13 or tg131 (Amersham) and transformation into JM101 (Messing and Vieira, (1982), Gene, 19, 269-276). The complete sequence of human TIMP and the predicted amino acid sequenceis shown in Figure 3. The numbers beneath the sequence refer to the sequence of mRNA. The nucleotides at positions 27 and 706 were not identified. The numbers above the sequence refer to the amino acid sequence, negative numbers refer to the putative signal sequence. Potential glycosylation sites are underlined.

## 2. NUCLEOTIDE SEQUENCE OF HUMAN TIMP cDNA AND THE PREDICTED AMINO ACID SEQUENCE (FIGURE 3)

The cDNA is 793 nucleotides in length and contains an open reading frame of 621 nucleotides (nucleotides 64-684 in Figure 3). The 3' end of the TIMP mRNA appears to be represented because it has a poly A sequence preceded by the polyadenylation signal AATAAA (Proudfoot and Brownlee, (1981), Nature, 252, 359-362). The nucleotides at positions 64-66 are thought to be at the start of translation since the methionine (M) that they encode is the only one in any reading frame that precedes the NH$_2$-terminal amino acids of
TIMP which are encoded by nucleotides 133-216.   The
sequence therefore translates into a polypeptide of
207 amino acids and terminates at the stop codon TGA
(nucleotides 685-687).   Nucleotides 196-198 predict
a lysine (K) at this position rather than the
leucine (L) reported by Stricklin and Welgus (J.
Biol. Chem., (1983), 258, 12252-12258).   However,
the NH$_2$-terminal amino acid sequence of human
amniotic fluid TIMP and TIMP purified from CCL153
culture supernatants does have a lysine at this
position (see Figure 1).   This suggests that the
DNA sequence is accurate and confirms that the cDNA
does encode human TIMP.   It also demonstrates the
close similarity of TIMP isolated from the CCL153
human diploid lung cell line, human amniotic fluid
and human skin fibroblasts.

Following the NH$_2$-terminal methionine encoded
by nucleotides 64-66 there are 22 amino acids, many
of which are hydrophobic, and which precede the
NH$_2$-terminal cysteine (C) found in both human
amniotic fluid TIMP and the fibroblast collagenase
inhibitor.   This is consistent with these amino
acids being the hydrophobic core of a signal
sequence which is cleaved during secretion to
liberate the mature protein.   Mature TIMP is
therefore 184 amino acids in length and has a
predicted molecular weight of 20,685.   The total
amino acid composition of mature TIMP predicted from
the DNA sequence is described in Table 1 below.   Of
interest are the twelve cysteine (c) residues in
mature TIMP which is half the number estimated by
Stricklin and Welgus (J. Biol. Chem., (1983),
258,12259-12264) to be present in the fibroblast
inhibitor, but is consistent with the cysteine
content reported for a bovine metalloproteinase
inhibitor (Kishi and Hayakawa, J. Biochem., 1984, 9
6, 395-404).

TABLE 1                                    0188312

Amino acid composition of mature human TIMP predicted from the cDNA sequence

| Residue | Number | % | Weight | % |
|---------|--------|------|--------|------|
| F | 10 | 5.41 | 1470 | 7.11 |
| L | 18 | 9.73 | 2034 | 9.83 |
| I | 7 | 3.78 | 791 | 3.82 |
| M | 3 | 1.62 | 393 | 1.90 |
| V | 9 | 4.86 | 891 | 4.31 |
| S | 13 | 7.03 | 1131 | 5.47 |
| P | 10 | 5.41 | 970 | 4.69 |
| T | 16 | 8.65 | 1616 | 7.81 |
| A | 11 | 5.95 | 781 | 3.78 |
| Y | 6 | 3.24 | 978 | 4.73 |
| H | 6 | 3.24 | 822 | 3.97 |
| Q | 12 | 6.49 | 1536 | 7.43 |
| N | 4 | 2.16 | 456 | 2.20 |
| K | 8 | 4.32 | 1024 | 4.95 |
| D | 5 | 2.70 | 575 | 2.78 |
| E | 9 | 4.86 | 1161 | 5.61 |
| C | 12 | 6.49 | 1236 | 5.98 |
| W | 3 | 1.62 | 558 | 2.70 |
| R | 10 | 5.41 | 1560 | 7.54 |
| G | 12 | 6.49 | 684 | 3.31 |

| Total | 184 | | 20,685 | |

Finally, the DNA sequence predicts two potential N-glycosylation sites in the mature protein and these are indicated by underlining in Figure 3.

## 3. THE PRODUCTION OF TIMP

### 3.1 Expression in E.coli

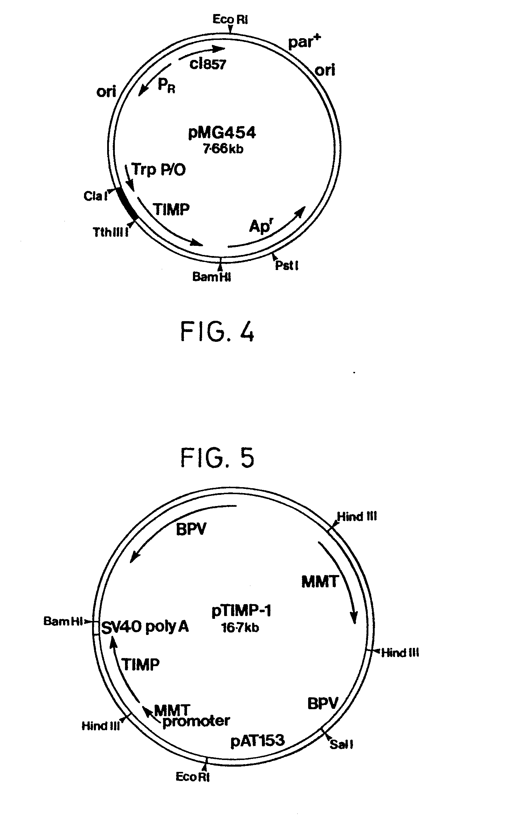

The expression of TIMP with an additional $NH_2$-terminal methionine residue (met-TIMP) in E.coli can be achieved by using oligonucleotide linkers to join the mature TIMP encoding sequence described in Section 2 above and shown in Figure 3 with a promoter, Shine Dalgarno sequence and an initiating ATG codon. In this example, the E.coli trpE promoter and Shine Dalgarno sequence was used. These sequences are present for example on plasmid pCT54 into which DNA sequences encoding proteins of interest can be inserted in a ClaI site (Emtage et al, (1983), Proc. Nat. Acad. Sci. USA, 80, 3671-3675). However, other plasmids also harbouring these or other promoter and Shine Dalgarno sequences but which are amplifiable and from which expression can be tightly regulated may also be used ( see, for example, European patent application EP-A2-0121386). In this example the TIMP coding sequence without the signal coding sequence but with an additional N-terminal methionine codon, was inserted into the E.coli expression vector pMG196 to produce pMG454 (Figure 4). This was achieved by standard recombinant DNA techniques (Maniatis et al, (1982), In: Molecular Cloning: A Laboratory Manual, 390-433). pMG454 exists stably in E.coli at low copy number in the absence of selection but on induction increases in copy number to 100-200 per chromosome.

For insertion into the expression vector the HinfI site (nucleotides 686-690) adjacent to the TIMP stop codon (Figure 3) was converted with a linker into a BamHI site.  The 5' end of the cDNA was then reconstructed from the TthIII-1 site located at nucleotides 178-186 (see Figure 3) using a 61 bp linker to provide an upstream ClaI site followed by an ATG start codon preceeding the cysteine codon at the N-terminus of mature TIMP. The sequence of this linker is as follows:

```
            M   C   T   C   V   P   P   H   P   Q   T   A   F   C   N   S   D
CGATAACTAATGTGTACCTGCGTTCCACCTCATCCTCAAACTGCTTTCTGCAACTCTGACC

TATTGATTACACATGGACGCAAGGTGGAGTAGGAGTTTGACGAAAGACGTTGAGACTGGA
```

Cla1                                                      TthIII-1

It was assembled by standard techniques from the following oligonucleotides:

```
CGATAACTAATGTGTACCTGCGT
TCCACCTCATCCTCAAAC
TGCTTTCTGCAACTCTGACC
CATTAGTTAT
TGAGGTGGAACGCAGGTACA
AGCAGTTTGAGGA
AGGTCAGAGTTGCAGAA
```

Each oligonucleotide was synthesised by automated solid phase phosphotriester chemistry ( Patel et al, (1982), Nuc. Acid Res., 10, 5605-5619).

The linker was used to ligate the 5' end of the TIMP gene via the TthIII 1 site located within the TIMP coding sequence with the ClaI site situated downstream from the trpE Shine Dalgarno sequence present in plasmid pMG196. The resulting plasmid, pMG454 was established by transformation into E.coli E103S (a proteinase-deficient E.coli K12 derivative). The result is a thirteen nucleotide sequence between the Shine Dalgarno sequence and the initiating ATG which is immediately adjacent to the N-terminal cysteine (C) of mature TIMP.

Cultures of E103S containing the plasmid were grown at $30^{\circ}C$ in L-broth to an OD600 of 0.4 and then shifted to $42^{\circ}C$ by shaking at $65^{\circ}C$. On reaching $42^{\circ}C$ they were transferred to a $37^{\circ}C$ water bath and grown for a further 5 hours when appropriately sized aliquots were taken and analysed by SDS gel electrophoresis and Western blotting . As shown in Figure 6, after induction a protein of $M_r$ 20,000 was observed, the expected size for unglycosylated TIMP. Note that β-lactamase (which is also encoded by pMG454) is also specifically induced. By Western blotting it was shown that the $M_r$ 20,000 protein reacts specifically with polyclonal antibodies directed against TIMP purified from human amniotic fluid (Figure 7: track 2). As with several other recombinant proteins expressed in E.coli, TIMP was found to be mostly in an insoluble form in inclusion bodies.

Improved TIMP yields were achieved by altering the DNA sequence encoding the N-terminal amino acids of Met-TIMP in pMG454. The new plasmids which resulted were generated essentially as described for the construction of pMG454 except that some of the oligonucleotides comprising the 61 bp linker had different sequences. Some of these changes result in new N-terminal amino acids. The resulting plasmids and TIMP 5' and N-terminal sequences are as follows:

|         |   M C T    |
|---------|------------|
| pMG454  | ATGTGTACC  |
|         |   M C I    |
| pMG456  | ATGTGTATC  |
|         |   M F T    |
| pMG457  | ATGTTTACC  |
|         |   M C T    |
| pMG468  | ATGTGTACT  |

Further improvements in yields of TIMP were achieved by altering the number of nucleotides between the Shine Dalgarno sequence and the ATG initiation codon in plasmid pMG454. This was undertaken by linearising pMG454 at the ClaI site, treating with Sl nuclease, and religating essentially as described Emtage et al ((1983), Proc. Natl. Acad. Aci., 80, 361-3675). Plasmid pMG461 was generated in this way.

Finally, the T7 transcription terminator sequence (Dunn and Studier, (1980), Nucl. Acid Res., $\underline{8}$, 219-2132) was placed in close proximity to the 3' end of the TIMP coding sequence in the best producing plasmid.   The sequence was ligated into the plasmid at the BamHI site .

## 3.2 Purification of TIMP from E.coli Cells

E.coli cells harbouring plasmid constructed as described above, when grown under optimal conditions for expression, produce met-TIMP at levels of up to 10% of total cellular protein.

The soluble protein fraction from crude E.coli extracts is assayed for TIMP activity essentially as described for conditioned media (Hicks et al, (1984), Int. J. Cancer, $\underline{33}$, 835-844).   A part or whole of the met-TIMP expressed in E.coli may be in an insoluble form and therefore not detected in the above assay.   In such circumstances, it is solubilised and activated prior to assay and purification.   One example of how this can be achieved in relation to methionine-prochymosin production is described in our co-pending International patent application PCT/GB 83/00152 (published as WO 83/04418) and in published British patent application GB2100737A.   Having obtained soluble, active, partially purified TIMP, it is further purified by immunoaffinity chromatography (see, for example, Eveleigh and Levy, (1977), J. Solid Phase Biochem., $\underline{2}$, 45) using the the sheep polyclonal antibodies described above or monoclonal antibodies raised from authentic TIMP purified from CCL153 cell supernatants. Alternatively, further purification is achieved using standard protein purification techniques, as described for example in Murphy et al, J. Biochem., *195*, 167-170 (1981) and Mercer *et al*, Biochem. J., *231*, 505-510 (1985).

### 3.3 Expression in Yeast

Using standard recombinant DNA techniques in a similar manner to those used to achieve expression in E.coli, plasmid vectors suitable for expression of TIMP in yeast are constructed, again using oligonucleotide linkers and the TIMP encoding cDNA described in Section 2 and shown in Figure 3. The constructions are based, for example, on the vectors described in co-pending published European patent application EP-A2-0073635. They contain the TIMP encoding sequence flanked by the yeast phosphoglycerate kinase (PGK) promoter and the PGK gene 3' untranslated end. The orientation of the TIMP cDNA with respect to the PGK promoter is such that it ensures expression of mature TIMP with an additional $NH_2$-terminal methionine residue as described for expression in E.coli. Alternatively, pre-TIMP may be expreseed by joining the TIMP encoding sequence to the vector such that the TIMP signal sequence is left in place. The Ncol site at nucleotides 32-37 (Figure 3) can be used for this purpose. Expression of a fusion protein between a yeast signal sequence (for example, the yeast ∝ -Factor signal sequence (Kurjan and Herskowitz, (1982), Cell, *30*, 933-943), and mature TIMP can be achieved through the use of appropriate linkers. These plasmid DNAs are introduced into yeast cells, for example, by the method of Beggs (Nature, (1978), *275*, 104-109).

### 3.4 Purification of TIMP from Yeast Cells

Yeast cells containing these plasmids, when grown under optimal conditions for TIMP expression, produce up to 5% of total cellular protein as TIMP. Depending on the alternatives described above, TIMP may or may not be secreted from the yeast cells. Expressed TIMP is quantified, assayed and purified essentially as described above if produced intercellularly. If secreted it is purified from cell supernatants as described above or by standard protein purification techniques (Murphy et al, J. Biochem., 195, 167-170 (1981) and Mercer et al, Biochem. J., 231, 505-510 (1985).

### 3.5 Expression of TIMP in Cultured Animal Cells

Expression of pre-TIMP in animal cells was achieved using vectors such as those described by Howley et al ((1983) United States patent specification 4419446) or other non-viral vectors. The necessary DNA manipulations were achieved using standard techniques (Maniatis et al, (1982), Cold Spring Harbor Laboratory, New York). The DNA sequence encoding pre-TIMP was excised from the cDNA described in Section 2 and shown in Figure 3 and the 3' Hinf1 site adjacent to the TIMP stop codon (nucleotides 686-690) was converted, with a linker, into a BamHI site. Similarly the 5' NcoI site adjacent the ATG start codon (nucleotides 62-67) was converted (after partial NcoI digestion since there is an internal NcoI site), with a linker, into a HindIII site. The entire pre-TIMP coding sequence was then inserted between the murine metallothioneine (MMT) promoter and the SV40 early transcript polyadenylation signal in the bovine papilloma virus based vector as shown in Figure 5.

This plasmid was named pTIMP-1.

After transfection of pTIMP into C127 cells (Lowy et al, J. Virol 26, 291-298, (1978)) by calcium phosphate co-precipitation (Graham and van der Eb, Virology, 52, 456-461, 1983) $CdCl_2$ (20µM) and $ZnCl_2$ (20 µM) resistance foci were selected. Their ability to secrete TIMP after 16 hour growth in serum-free medium was measured by a competitive RIA based on immobilised specific antibodies to TIMP (Hembry, Murphy & Reynolds, J. Cell Sci., 73, 105-119, (1985)), Western blotting and with the collagen fibril assay as described for the CCL153 cell supernatants. The RIA utilised polyclonal antibodies directed against purified human amniotic fluid TIMP and which are known not to cross-react with bovine or murine TIMP (Hembry, Murphy & Reynolds, J. Cell Sci., 73, 105-119, (1985)) detected a 200-fold increase in some of the pTIMP-1 transfected cell lines compared to control cells transfected with plasmid without inserted cDNA. Highest yields of up to 1.5 - 2.0 µg $ml^{-1}$ were obtained.

Additionally, Western blotting indicated that the TIMP secreted by the transfected C127 cells had a $M_r$ identical to authentic TIMP purified from human amniotic fluid, suggesting that in these cells the recombinant TIMP is correctly processed and glycosylated (Figure 7: track 4). To see whether the immunoreactive TIMP made in the C127 cells was biologically active, the culture supernatants were tested for their ability to inhibit rabbit skin collagenase using the collagen fibril assay (Murphy et al, J. Biochem., 195, 167-170, (1981)). No activity was detected in the supernatants of those cells shown in the RIA to be making undetectable amounts of TIMP. However, 2-5U$ml^{-1}$ was detected in

the supernatants of those cells shown in the RIA to be making in excess of 500 ng ml$^{-1}$ TIMP, indicating that pTIMP-1 directs the synthesis of active TIMP in C127 cells.   Further refinements of pTIMP-1 harbouring cell lines have provided yields of active TIMP in excess of 5$\mu$gml$^{-1}$.

## 3.6 Purification of TIMP from Animal Cells

Pre-TIMP, expressed in animal cells as outlined above, is secreted through the cellular membrane where cleavage of the signal peptide occurs to release mature TIMP.  It may be assayed and purified from the tissue culture media as described in Section 3.2 above.

## 4. Inhibition of Lysis of Collagen Films by Rabbit Tumour Cells: Comparison of Recombinant TIMP and Natural TIMP

Human fibroblast TIMP was purified and recombinant human TIMP expressed in mammalian cells was partially purified by heparin Sepharose and gel filtration chromatography (Murphy, G., et al, (1981), Biochem. J., 195, 167-170).   The recombinant material was comparable with natural human TIMP as defined by biochemical and ELISA assays.

To test biological activity primary VX2 rabbit tumour cells ($10^5$/well) were passaged onto $^{14}$C-labelled type I collagen films.

Materials.  Human plasminogen (20 casein units/mg) was purchased from Kabi Diagnostica, Stockholm, Sweden.   All other materials were previously described (Murphy, G., Cawston, T.E. and Reynolds, J.J. (1981) Biochemical Journal 195, 167-170. Hembry, R.M., Murphy, G., Cawston, T.E., Dingle, J.T.

and Reynolds, J.J. (1985) Journal of Cell Science, in press., Hembry, R.M., Murphy, G. and Reynolds, J.J. (1985), Journal of Cell Science 73, 105-119, Sellers, A. and Reynolds J.J. (1977), Biochemical Journal 167, 353-360, Trechsel, U., Dew, G., Murphy, G. and Reynolds, J.J. (1982), Biochimica Biophysica Acta 720, 364-370).

VX2 Tumour Cells. The rabbit VX2 carcinoma was originally induced by Kidd and Rouse (Kidd, J.G., and Rous, P. (1940), Journal of Experimental Medicine $\underline{71}$, 813-837) with Shope papilloma virus but is now virus-free. Our original tumour was kindly provided by Professor C.S.B. Galasko and colleagues (Department of Orthopaedic Surgery, University of Manchester, Hope Hospital, Eccles Old Road, Salford, U.K.) and was carried by serial transplantation in the thigh muscle of New Zealand White rabbits. It consistently metastasised to the lungs. After two to three weeks post-implantation of the tumour the animals were killed and the tumour excised. Primary tumour cells were derived by teasing cells out of the tumour and plating them at $0.2 \times 10^6$ cells/cm$^2$ in Dulbecco's Modification of Eagle's Medium (DMEM) supplemented with 10% fetal calf serum (FCS). The primary tumour cells were passaged onto collagen films after 7-10 days using 0.02% EDTA in $Ca^{2+}/Mg^{2+}$-free phosphate buffered saline (PBS-A). First passage tumour cells ($0.5 \times 10^6$) formed a palpable tumour 4 weeks after injection into the thigh muscle of a rabbit.

Collagen Films. Radiolabelled collagen films were prepared using a modification of the method of Johnson-Wint (Johnson-Wint, B. (1980), Analytical Biochemistry, 104, 175-181). Aliquots (300ul) of

$^{14}$C-labelled type I collagen (rat skin, 150µg) in 10mM phosphate, 300mM sodium chloride, 0.02% azide were plated in 2cm diameter Linbro wells. The plates were allowed to dry at 37°C and then washed twice with sterile distilled water and once with serum-free medium. Cells were plated onto the washed film at 10$^5$/well in DMEM containing 10% FCS. After 24h the cell layers were washed gently with serum-free medium and DMEM containing 2% rabbit serum (selected for negligible levels of proteinase inhibitors; Trechsel et al (1982) was added to each well. After 2 days medium was collected from each well, spun to remove undigested collagen and radioactivity released from the film was estimated by liquid scintillation counting. Undegraded collagen remaining in the wells was digested with 50ug bacterial collagenase overnight and the radioactivity estimated. From these data the % release of radioactivity from each of the films was calculated. The susceptibility of the collagen films to non-specific degradation was assessed by incubating the collagen films with 10µg trypsin/well. The radioactivity released was 15-20%, comparable to similar trypsin blanks performed in a conventional fibril assay. Certain wells were treated either with a specific polyclonal antibody raised in sheep against purified rabbit bone collagenase (Hembry, et al, loc. cit. with normal sheep serum IgG.

Assays for Collagenase Activity and TIMP.
Collagenase and TIMP assays were performed as previously described (Sellers and Reynolds, (1977) loc. cit. 1 unit of collagenase degrades 1µg of collagen per min at 35°C. TIMP was assayed by the inhibition of activated rabbit collagenase; 1 unit is defined as the amount of TIMP which inhibits 2 units of collagenase by 50%.

After 2 days, culture media were changed to media with serum that had low inhibitory activity towards metalloproteinases. Then either control buffer, or pure human fibroblast TIMP (2 units/well), or partially purified recombinant TIMP (2 units/well) was included in the medium for a further two day culture period. Aliquots of conditioned media were counted in a scintillation counter to assess gel lysis and the inhibition was calculated as shown in Table 2 and Figure 8. Table 2 illustrates two experiments to show the similarity between the action of the natural and recombinant TIMP, and Figure 8 illustrates another experiment with recombinant TIMP alone. (In Figure 8 the results show the mean values of three experiments; ▥ with partially purified recombinant TIMP (2 units/well) and ▧ without recombinant TIMP).

| | Counts/well | % Inhibition |
|---|---|---|
| **Experiment 1** | | |
| Control buffer | 3550 ± 280 | |
| Pure human TIMP | 709 ± 76 | 80 |
| **Experiment 2** | | |
| Control buffer | 8456 ± 1035 | |
| Recombinant TIMP | 764 ± 29 | 91 |

It will be understood that the invention is described above by way of example only and modifications of detail within the scope of the invention may be made.

CLAIMS:

1. A process for the production of a metalloproteinase inhibitor comprising culturing host cells transformed with a vector including a gene coding for the metalloproteinase inhibitor.

2. A process according to claim 1 wherein the metalloproteinase inhibitor is a methionine-metalloproteinase inhibitor.

3. A process for the production of a metalloproteinase inhibitor comprising producing a precursor of the metalloproteinase inhibitor by culturing host cells transformed with a vector including a gene coding for the metalloproteinase inhibitor precursor and cleaving the precursor to produce the metalloproteinase inhibitor.

4. A process according to claim 3 wherein the metalloproteinase inhibitor precursor is a premetalloproteinase inhibitor and the host cells are host cells capable of cleaving the premetalloproteinase inhibitor to produce the metalloproteinase inhibitor.

5. A process according to claim 3 wherein the metalloproteinase inhibitor precursor is a fusion protein comprising a heterologous protein and a metalloproteinase inhibitor protein.

6. A methionine-metalloproteinase inhibitor.

7. A premetalloproteinase inhibitor.

8. A fusion protein comprising a heterologous protein and a metalloproteinase inhibitor.

9. A gene coding for the amino acid sequence of a metalloproteinase inhibitor, a methionine-metalloproteinase inhibitor, a premetalloproteinase inhibitor or a fusion protein comprising a heterologous protein and a metalloproteinase inhibitor.

- 35 -

0188312

10. A gene according to claim 9 having substantially the nucleotide sequence from 64 to 684 inclusive as shown in Figure 3 of the accompanying drawings.

11. A gene according to claim 9 having substantially the nucleotide sequence from 133 to 684 inclusive as shown in Figure 3 of the accompanying drawings.

12. A vector including a gene according to any one of claims 9 to 11.

13. Host cells transformed with a vector according to claim 12.

14. A pharmaceutical composition comprising a metalloproteinase inhibitor and a pharmaceutically acceptable excipient.

15. Plasmid pMG454, pMG456, pMG457, pMG468 or pMG461 and derivatives thereof.

16. Plasmid pTIMP-1 and derivatives thereof.

a.    C T C V P P H P Q T A F C N S D L V I R A K F V G T P E
b.    C T C V P P H (P) Q T A F C N (S) D L V I R A L F V G T P E

```
                 C   A          A     G           T  C     C  C          AA
c.  5'- TGCACCTGTGTGCCCCCCCACCCCCAGACAGCCTTCTGCAACTCTGACCTGGTGATCAGGGCCCTGTTC -3'
           140      150       160       170       180      190       200
```

```
                    C              A
d.        3'- GTCTGTCGGAAGACGTTG -5'
```

## FIG. 1

## FIG. 2

<----------------------------SIGNAL SEQUENCE---------------
                                        -20                                                    -10
                                        M  A  P  F  E  P  L  A  S  G  I  L  L  L  L  W  L  I  A
AGGGGCCTTAGCGTGCCGCATCGCCG-AGATCCAGCGCCCAGAGAGAGACACCAGAGAACCCACCATGGCCCCCTTTGAGCCCCTGGCTTCTGGCATCCTGTTGTTGCTGTGGCTGATAGCC
        10        20        30        40        50        60        70        80        90        100       110       120
------------>

            -1  1                              10                        20       *                    30
P  S  R  A  C  T  C  V  P  P  H  P  Q  T  A  F  C  N  S  D  L  V  I  R  A  K  F  V  G  T  P  E  V  N  Q  T  T  L  Y  Q
CCCAGCAGGGCCTGCACCTGTGTCCCACCCCACCCACAGACGGCCTTCTGCAATTCCGACCTCGTCATCAGGGCCAAGTTCGTGGGGACACCAGAAGTCAACCAGACCACCTTATACCAG
        130       140       150       160       170       180       190       200       210       220       230       240

        40                              50                        60                        70
R  Y  E  I  K  M  T  K  M  Y  K  G  F  Q  A  L  G  D  A  A  D  I  R  F  V  Y  T  P  A  M  E  S  V  C  G  Y  F  H  R  S
CGTTATGAGATCAAGATGACCAAGATGTATAAAGGGTTCCAAGCCTTAGGGGATGCCGCTGACATCCGGTTCGTCTACACCCCCGCCATGGAGAGTGTCTGCGGATACTTCCACAGGTCC
        250       260       270       280       290       300       310       320       330       340       350       360

        80                              90                        100                       110
H  N  R  S  E  E  F  L  I  A  G  K  L  Q  D  G  L  L  H  I  T  T  C  S  F  V  A  P  W  N  S  L  S  L  A  Q  R  R  G  F
CACAACCGCAGCGAGGAGTTTCTCATTGCTGGAAAACTGCAGGATGGACTCTTGCACATCACTACCTGCAGTTTCGTGGCTCCCTGGAACAGCCTGAGCTTAGCTCAGCGCCGGGGGCTTC
        370       380       390       400       410       420       430       440       450       460       470       480

        120                             130                       140                       150
T  K  T  Y  T  V  G  C  E  E  C  T  V  F  P  C  L  S  I  P  C  K  L  Q  S  G  T  H  C  L  W  T  D  Q  L  L  Q  G  S  E
ACCAAGACCTACACTGTTGGCTGTGAGGAATGCACAGTGTTTCCCTGTTTATCCATCCCCTGCAAACTGCAGAGTGGCACTCATTGCTTGTGGACGGACCAGCTCCTCCAAGGCTCTGAA
        490       500       510       520       530       540       550       560       570       580       590       600

        160                             170                       180
K  G  F  Q  S  R  H  L  A  C  L  P  R  E  P  G  L  C  T  W  Q  S  L  R  S  Q  I  A
AAGGGCTTCCAGTCCCGTCACCTTGCCTGCCTGCCTCGGGAGCCAGGGCTGTGCACCTGGCAGTCCCTGCGGTCCCAGATAGCCTGAATCCTGCCCGGAGTGGAA-CTGAAGCCTGCACA
        610       620       630       640       650       660       670       680       690       700       710       720

GTGTCCACCCTGTTCCCACTCCCATCTTTCTTCCGGACAATGAAATAAAGAGTTACCACCCAGCAAAAAAAAA
        730       740       750       760       770       780       790

FIG. 3

FIG. 4

FIG. 5

FIG. 6

FIG. 7

FIG. 8